# EUROPEAN PATENT APPLICATION

(11) **EP 4 667 932 A1**
(43) Date of publication of application: **24.12.2025**
(21) Application number: 24183195.7
(22) Date of filing: 19.06.2024
(51) Int. Cl.: G01N 33/00

(54) **TANK MONITORING DEVICE AND METHOD OF EVALUATING CHEMICAL COMPATIBILITY OF PRODUCTS CONTAINED IN TANKS**

(71) Applicant: Rosemount Tank Radar AB, 435 23 Mölnlycke (SE)
(72) Inventor: WIDAHL, Christoffer, 426 70 Västra Frölunda (SE); LENNING, Erik, 416 55 Göteborg (SE)
(74) Representative: Kransell & Wennborg KB

(57) **Abstract**

A tank monitoring device (9a) for monitoring a first tank (5a) containing a first product, the tank monitoring device (9a) comprising a memory (24) configured to store chemical compatibility information about the first product; wireless communication circuitry (25); and a controller (27) configured to: receive, using the wireless communication circuitry (25), from a tank monitoring device (9b) monitoring a second tank (5b), chemical compatibility information about a second product contained in the second tank (5b); retrieve, from the memory (24), the chemical compatibility information about the first product; determine, based on the chemical compatibility information about the first product and the chemical compatibility information about the second product, that the first product and the second product are chemically incompatible; and provide an indication that the first product and the second product are chemically incompatible.

## Description

### Technical Field of the Invention

The present invention relates to a tank monitoring device, and to a method of evaluating a chemical compatibility of products contained in tanks.

### Technical Background

Tanks containing chemically incompatible products should not be stored close to each other. Currently, markings on the tank and operators making decisions regarding chemical compatibility based on such markings are generally relied on. This, however, leaves room for human error. Additionally, exposure to sunlight, rain, or rough handling, etc. may make such markings unreadable.

It would be desirable to provide for improved evaluation of the chemical compatibility of products contained in tanks.

### Summary

In view of the above, a general object of the present invention is to provide for improved evaluation of the chemical compatibility of products contained in tanks.

According to a first aspect of the present invention, it is therefore provided a tank monitoring device for monitoring a first tank containing a first product, the tank monitoring device comprising: a memory configured to store chemical compatibility information about the first product contained in the first tank; wireless communication circuitry for wireless communication; and a controller for controlling operation of the tank monitoring device, the controller being configured to: receive, using the wireless communication circuitry, from a tank monitoring device monitoring a second tank, chemical compatibility information about a second product contained in the second tank; retrieve, from the memory, the chemical compatibility information about the first product contained in the first tank; determine, based on the chemical compatibility information about the first product and the chemical compatibility information about the second product, that the first product and the second product are chemically incompatible; and provide an indication that the first product contained in the first tank and the second product contained in the second tank are chemically incompatible.

The chemical compatibility information stored in the memory, when the tank monitoring device is in use, may, for example, be an indication of the product type and/or a coded product identifier.

The present invention is based on the realization that many of the issues of the current way of evaluating chemical compatibility of products contained in tanks can be solved by providing the tanks with tank monitoring devices capable of independently performing the evaluation of the chemical compatibility. The present inventors have further realized that this can be achieved by a tank monitoring device that can wirelessly receive chemical compatibility information from another tank monitoring device within a nearzone. This provides for automatic evaluation of the chemical compatibility of the products contained in the tanks, without having to rely on the human factor or visual markings on the tanks, and also allows for an indication, such as a warning, to be provided to an operator and/or to an asset management system, so that appropriate action can be taken promptly. An example of an appropriate action may be to move the second tank away from the first tank.

In an example of the tank monitoring device according to the present invention, the wireless communication circuitry may comprise first wireless communication circuitry configured to implement a first communication architecture adapted for relatively long range wireless communication; and second wireless communication circuitry configured to implement a second communication architecture adapted for relatively short range wireless communication, with a shorter range than when the first wireless communication circuitry is used.

The first wireless communication circuitry may require more power for operation than the second wireless communication circuitry. It may therefore be more important to minimize the active time for the first wireless communication circuitry than to minimize the active time for the second wireless communication circuitry. For instance, the first wireless communication circuitry may be active only when activated by (part of) the controller, and the second wireless communication circuitry may always be active, or may be activated considerably more frequently than the first wireless communication circuitry. According to an example, the second wireless communication circuitry may be active whenever the tank monitoring device is moving.

In an example of the tank monitoring device according to the present invention, the controller may be configured to receive the chemical compatibility information about the second product contained in the second tank using the second wireless communication circuitry. This allows the controller to receive the chemical compatibility information about the second product contained in the second tank more frequently, since the second wireless communication circuitry requires relatively low power. Hereby, the chemical compatibility of products in tanks that are close to each other can be evaluated more frequently, which may be beneficial to the safety where the tanks are stored or transported together.

In an example of the tank monitoring device according to the present invention, the controller may be configured to transmit the chemical compatibility information about the first product contained in the first tank. This allows evaluation of the chemical compatibility also by other tank monitoring devices, which may be arranged to monitor other tanks. In examples configurations where the wireless communication circuitry comprises the above-mentioned first and second wireless communication circuitries, the controller may be configured to transmit the chemical compatibility information using the second wireless communication circuitry. As explained above, this may enable more frequent transmission of the chemical compatibility information.

According to examples, the controller may be configured to broadcast the chemical compatibility information about the first product contained in the first tank. A benefit of this may be that other tank monitoring devices, arranged to monitor other tanks, can receive and evaluate the chemical compatibility information about the first product contained in the first tank without the need for first establishing communication with the tank monitoring device arranged to monitor the first tank.

The controller may be configured to broadcast the chemical compatibility information about the first product contained in the first tank at least once every hour. This may be considerably more frequently than wireless communication between the tank monitoring device and an asset management system, which may take place using the first wireless communication circuitry. Advantageously, the controller of the tank monitoring device may be configured to broadcast the chemical compatibility information about the first product more often, such as every five minutes, several times per minute, or continuously. For example, the controller may be configured to start to broadcast the chemical compatibility information about the first product contained in the first tank when the first tank (and thus the tank monitoring device) is in motion. Such more frequent, or even continuous broadcasting of the chemical compatibility information may reduce the risk that tanks containing chemically incompatible products are placed in the vicinity of each other, since an operator could be alerted when in the process of moving a tank with an incompatible product.

According to examples of the tank monitoring device of the present invention, the controller may be configured to receive, from an asset management system using the first wireless communication circuitry, the chemical compatibility information about the first product contained in the first tank. This may provide for keeping information about the first product up to date, and may obviate the need for manual intervention on site.

According to examples of the tank monitoring device of the present invention, the controller may be configured to transmit, to the asset management system using the first wireless communication circuitry, a message indicating that the first product contained in the first tank and the second product contained in the second tank are chemically incompatible. This may allow the asset management system to alert a remote operator of the chemical incompatibility, increasing the probability that appropriate action is taken. Furthermore, the asset management system can save the chemical incompatibility report in memory, advantageously with a time stamp, location, and tank identities, to provide a so-called audit trail.

According to examples of the tank monitoring device of the present invention, the memory may be configured to additionally store a set of chemical compatibility rules, and the controller may be configured to determine that the first product and the second product are chemically incompatible additionally based on the set of chemical compatibility rules. For example, the set of chemical compatibility rules may embody a compatibility table indicating incompatible product types.

According to examples of the tank monitoring device of the present invention, the controller may be configured to receive, using the wireless communication circuitry, a unique identifier of the second tank; and provide the indication that the first product contained in the first tank and the second product contained in the second tank are chemically incompatible, including the unique identifier of the second tank.

According to examples of the tank monitoring device of the present invention, the tank monitoring device may further comprise level sensing circuitry for measuring the level of the first product contained in the first tank, and the controller may be configured to control the level sensing circuitry to measure the level of the first product; and transmit, using the wireless communication circuitry, a message indicative of the measured level of the first product to an asset management system.

The level sensing circuitry comprised in the tank monitoring device may advantageously comprise transceiver circuitry configured to generate a transmit signal, transmit the transmit signal, and receive a reflection signal, and sensing processing circuitry configured to determine the level of the product based on a timing relation between the transmit signal and the reflection signal. The transmit signal may, for example be an electromagnetic signal (such as radar or lidar) or an acoustic signal (such as ultrasonic).

According to a second aspect of the invention, there is provided a method of evaluating a chemical compatibility of products contained in tanks, the method comprising: receiving, by wireless communication circuitry of a first tank containing a first product, chemical compatibility information about a second product contained in a second tank; retrieving, by processing circuitry of the tank, chemical compatibility information about the first product contained in the first tank from a memory of the first tank; determining, by the processing circuitry, based on the chemical compatibility information about the first product and the chemical compatibility information about the second product, that the first product contained in the first tank and the second product contained in the second tank are chemically incompatible; and providing, by the processing circuitry, an indication that the first product contained in the first tank and the second product contained in the second tank are chemically incompatible.

According to examples of the method of the present invention, the method may comprise receiving, by the wireless communication circuitry of the first tank, the chemical compatibility information about the second product contained in the second tank when the first tank is within a maximum range of corresponding wireless communication circuitry of the second tank. This may allow automatic evaluation of the chemical compatibility of products contained in tanks within a predefined distance from the first tank, corresponding to the maximum range of the wireless communication circuitry of the second tank.

In summary, the present invention thus relates to a tank monitoring device for monitoring a first tank containing a first product, the tank monitoring device comprising a memory configured to store chemical compatibility information about the first product; wireless communication circuitry; and a controller configured to: receive, using the wireless communication circuitry, from a tank monitoring device monitoring a second tank, chemical compatibility information about a second product contained in the second tank; retrieve, from the memory, the chemical compatibility information about the first product; determine, based on the chemical compatibility information about the first product and the chemical compatibility information about the second product, that the first product and the second product are chemically incompatible; and provide an indication that the first product and the second product are chemically incompatible.

### Brief Description of the Drawings

These and other aspects of the present invention will now be described in more detail, with reference to the appended drawings showing embodiments of the invention, wherein:
Fig 1 is a schematic illustration of geographically distributed tanks and an asset management system keeping records of the status of each tank and creating work orders for filling and/or moving tanks as needed;
Fig 2 schematically shows an exemplary tank with a tank monitoring device installed;
Fig 3 is a schematic illustration of an example configuration of a tank monitoring device;
Fig 4 is a schematic illustration of an example configuration of a tank monitoring device;
Fig 5 is a schematic illustration of an example configuration of a tank monitoring device;
Fig 6 is an exemplary illustration of an operator tasked with fulfilling a work order at a site with multiple tanks;
Fig 7 is a flowchart illustrating an example method;
Fig 8 illustrates chemical compatibility evaluation according to an example; and
Fig 9 illustrates chemical compatibility rules according to an example.

### Detailed Description of Example Embodiments of the Invention

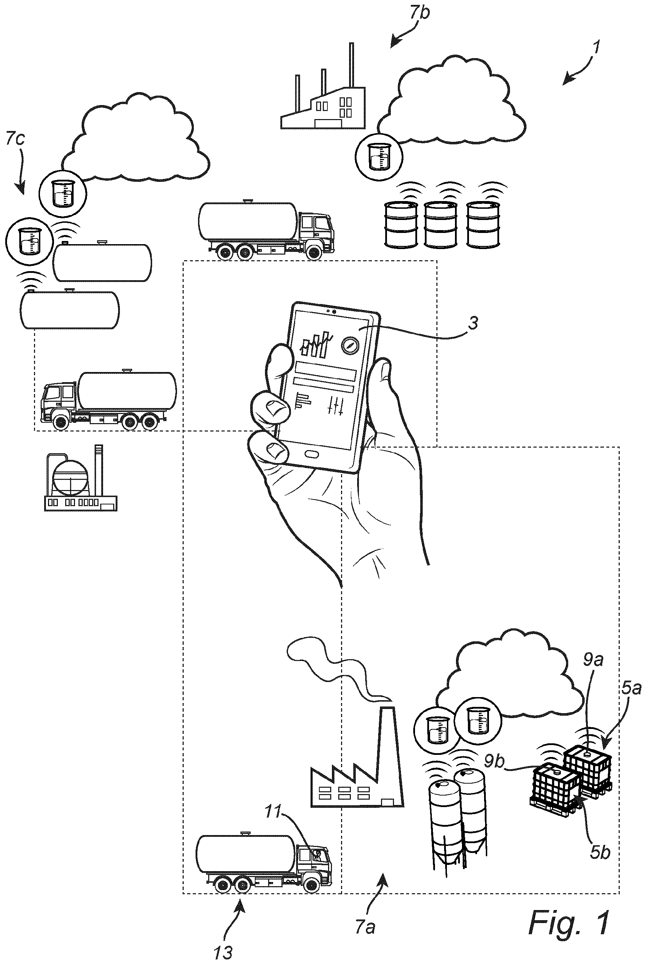

Fig 1 is a schematic illustration of an asset management system 1, and assets that may be managed by the asset management system 1. The asset management system 1 is here embodied in the form of software running on servers (represented by the clouds in fig 1) and on client devices (represented by a mobile device 3 of an asset management system operator in fig 1). Assets are represented by tanks 5a-b (only two of the tanks are provided with reference numerals in fig 1 to avoid cluttering the drawing) grouped at different locations 7a-c. The asset management system 1 may, for example, be used by a product availability provider. To keep track of the tanks 5a-b and their contents, the asset management system 1 may periodically receive updated information about the status of each tank 5a-b from a tank monitoring device 9a-b attached the tank 5a-b. In example configurations, a tank monitoring device 9a-b may be capable of measuring the level of product in its tank 5a-b, enabling the asset management system 1 to ensure that the tank 5a-b always contains a minimum amount of product. When the updated measured level of product in a tank 5a-b indicates that the tank should soon be refilled, the asset management system 1 may create a work order and provide that work order to an operator 11. The operator 11 may then bring a mobile vessel 13 (such as a tanker truck) with the relevant product to the location 7a-c of the tank 5a-b, and fill the tank 5a-b with the correct product for that tank. Tanks 5a-b may also be moved between locations 7a-c and within locations, depending on the type of tank.

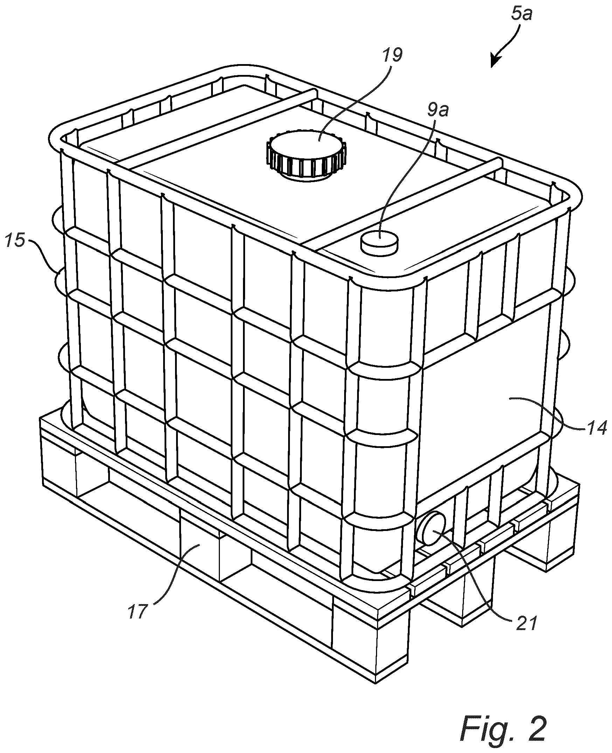

Fig 2 schematically shows an exemplary tank 5a (in this case a so-called IBC-tank) with a tank monitoring device 9a installed. Referring to fig 2, the IBC-tank 5a comprises a plastic container 14, a metal cage 15, and a pallet 17. The plastic container 14 is provided with a top opening 19 for filling product and a bottom opening 21 for emptying product. As is schematically illustrated in fig 2, the IBC-tank 5a is further provided with the above-mentioned tank monitoring device 9a that may, in this particular configuration, be attached to the IBC-tank 5a for measuring the level of product in the tank through the wall of the plastic container 14.

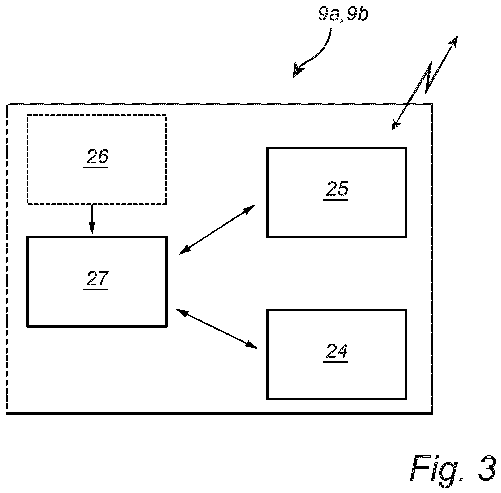

Fig 3 is a schematic illustration of a tank monitoring device 9a, 9b according to an example. Referring to fig 3, the tank monitoring device 9a, 9b comprises a memory 24, wireless communication circuitry 25, and a controller 27. Optionally, the tank monitoring device 9a, 9b may additionally comprise positioning circuitry 26, such as GPS-circuitry. In this first example configuration, the memory 24 may be configured to store chemical compatibility information about a first product contained in the tank 5a, 5b, to which the tank monitoring device 9a, 9b is coupled, such as attached. The controller 27 is coupled to the memory 25, the wireless communication circuitry 25, and, optionally, the positioning circuitry 26. The controller 27 is configured to receive, using the wireless communication circuitry 25 chemical compatibility information about a second product contained in a second tank, retrieve, from the memory 24, the chemical compatibility information about the first product; determine, based on the chemical compatibility information about the first product and the chemical compatibility information about the second product, whether the first product and the second product are chemically incompatible, and provide, when the result of the evaluation is that the first and second products are chemically incompatible, an indication that the first product contained in the first tank and the second product contained in the second tank are chemically incompatible. The indication may be provided locally, using an indicator (not shown in fig 3) comprised in the tank monitoring device 9a, 9b. Such an indicator may be configured to provide a visual indication and/or an audible indication, for example. Additionally, the indication may be provided to the asset management system, using the wireless communication circuitry 25.

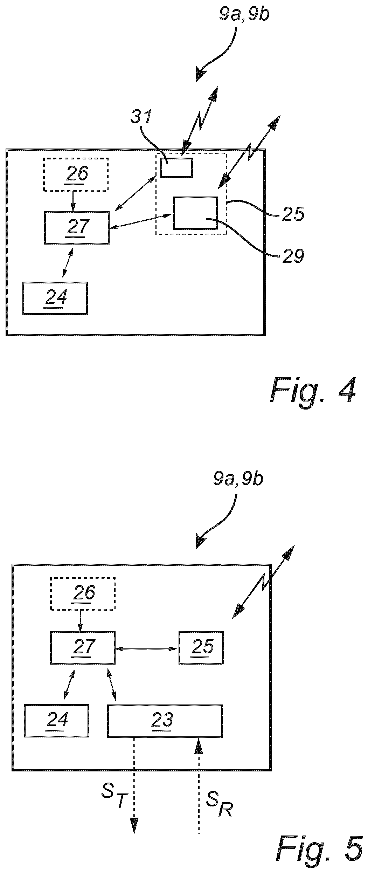

Fig 4 is a schematic illustration of a tank monitoring device 9a, 9b according to an example. In this example configuration, the wireless communication circuitry 25 comprises first wireless communication circuitry 29 configured to implement a first communication architecture adapted for relatively long range wireless communication; and second wireless communication circuitry 31 configured to implement a second communication architecture adapted for relatively short range wireless communication.

The first wireless communication circuitry 29 may thus be capable of wireless communication at a range that is beyond the capabilities of the second wireless communication circuitry 31. On the other hand, the second wireless communication circuitry 31 may require less power than the first communication circuitry, and the use of the second wireless communication circuitry 31 may be free.

The first wireless communication architecture may use at least one gateway for communication with the asset management system 1. Examples of the first wireless communication architecture may include LoRaWAN, Sigfox, Dash7, NB (Narrow Band) - IoT, and GSM etc. The second wireless communication architecture may rely on close range direct wireless communication. One suitable example of the second wireless communication architecture is BLE (Bluetooth Low Energy).

The controller 27 may be configured to receive the above-mentioned chemical compatibility information using the second wireless communication circuitry 31. The controller 27 may also be configured to transmit the chemical compatibility information about the first product in the tank to which the tank monitoring device 9a, 9b is coupled, using the second wireless communication circuitry 31. In particular, the controller may advantageously be configured to broadcast the chemical compatibility information about the first product, using the second wireless communication circuitry 31. This allows other tank monitoring devices within the range of the second wireless communication circuitry 31 to evaluate chemical compatibility in relation to the first product. As was mentioned above, the controller 27 of the tank monitoring device 9a, 9b may communicate with the asset management system using the first wireless communication circuitry 29. For example, the controller 27 may be configured to receive the chemical compatibility information about the first product from the asset management system using the first wireless communication circuitry 29, and store the received chemical compatibility information in the memory 24.

Fig 5 is a schematic illustration of a tank monitoring device 9a, 9b according to an example. In this example configuration, the tank monitoring device 9a, 9b comprises level sensing circuitry 23 for measuring the level of product in the tank 5a, 5b, to which the tank monitoring device 9a, 9b is coupled. In this example configuration, the controller 27 may be configured to control the level sensing circuitry 23 to measure the level of the product; and transmit a message indicative of the measured level to an asset management system, using the wireless communication circuitry 25.

The level sensing circuitry 23 may be configured to generate a transmit signal S_{T}, transmit the transmit signal S_{T} towards a surface of the product in the tank 5a, 5b, and receive a reflection signal S_{R} resulting from reflection of the transmit signal S_{T} at the surface of the product. The level sensing circuitry 23 may further be configured to determine the level of the product in the tank 5a, 5b based on a timing relation between the transmit signal S_{T} and the reflection signal S_{R}.

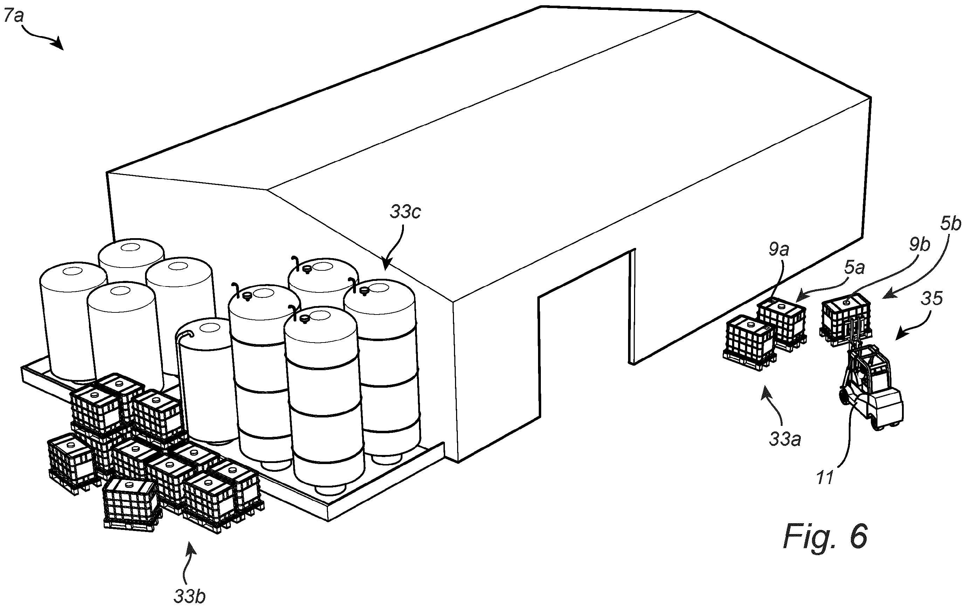

Fig 6 is an exemplary illustration, showing an operator 11, who may have been tasked by the asset management system 1 with fulfilling a work order at a site 7a with multiple tanks. At the example site 7a in fig 6, there are two groups 33a-b of IBC-tanks, and one group 33c of fixed storage tanks. In one of the groups 33a, which will be used to illustrate example embodiments of the present invention, the operator 11 is illustrated as being in the process of placing a second tank 5b in the vicinity of a first tank 5a, using a fork lift 35.

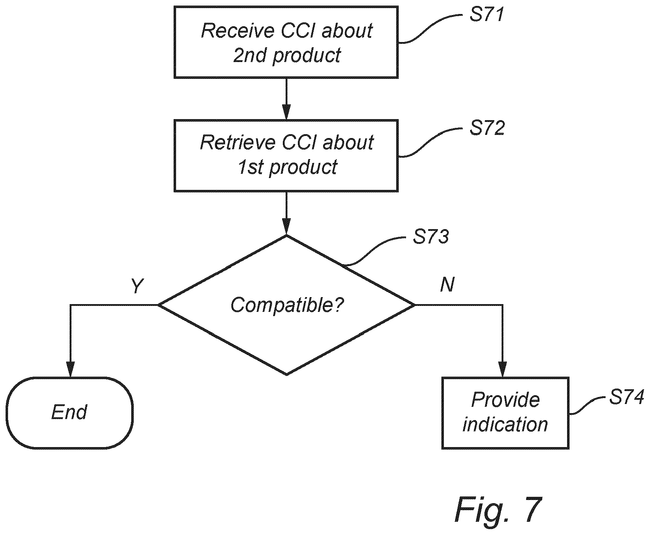

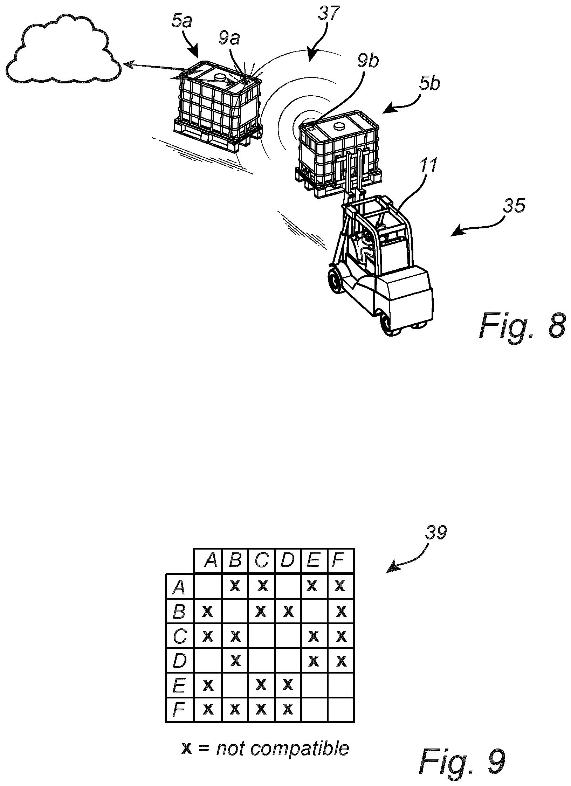

With this illustrative situation in mind, fig 7 shows an example method, fig 8 illustrates chemical compatibility evaluation according to an example, and fig 9 illustrates chemical compatibility rules according to an example.

Referring first to fig 7, wireless communication circuitry 25 of a first tank 5a receives S71 chemical compatibility information about a second product contained in the second tank 5b. The first tank 5a contains a first product.

Referring briefly to the illustration in fig 8, the tank monitoring device 9b of the second tank 5b may transmit, for example by broadcasting, as is indicated by the schematic radio waves 37 in fig 8, chemical compatibility information about the second product contained in the second tank 5b. The transmission may be by the above-mentioned second wireless communication circuitry 31 (with additional reference to the illustration in fig 4), which may allow the transmission to take place substantially continuously, or at least relatively often, such as at least once every hour, or at least once every five minutes, or every minute. Alternatively, or in combination, the controller 27 of the tank monitoring device 9b may control the wireless communication device 25 to transmit, for example by broadcasting, the chemical compatibility information about the second product in the second tank 5b in response to detecting that the second tank 5b is moving. Such detection may, for example, take place using the optional positioning circuitry 26 in figs 3-5, or using another motion detection device, such as an accelerometer or similar.

In examples where the chemical compatibility information about the second product in the second tank 5b is transmitted by the tank monitoring device 9b of the second tank 5b using the above-mentioned second wireless communication circuitry 31, the chemical compatibility information about the second product may be received by the second wireless communication circuitry 31 of the tank monitoring device 9a of the first tank 5a. This may occur as soon as the second wireless communication circuitry of the tank monitoring device 9a of the first tank 5a is within the range of the second wireless communication circuitry of the tank monitoring device 9b of the second tank 5b.

Again referring to the flow-chart in fig 7, the controller 27 of the tank monitoring device 9a of the first tank 5a retrieves S72 chemical compatibility information about the first product in the first tank 5a, from the memory 24. In addition to the chemical compatibility information, the controller can retrieve a set of chemical compatibility rules. Fig 9 illustrates an example of such a set of chemical compatibility rules, in the form of a table 39. The table 39, or other form of rule set, may, for example, be received from the asset management system 1, and be stored in the memory 24 of the tank monitoring device 9a.

The table 39 in fig 9 indicates the chemical compatibility of a first product having one or more of a number of basic chemical properties A-F, and a second product having one or more of a number of basic chemical properties A-F. The first product may be represented by the columns of the table 39, and the second product may be represented by the rows of the table 39. Chemically incompatible combinations of the basic chemical properties A-F are marked by 'X' in the table 39. Examples of the basic chemical properties A-F may be:

| | |
|---|---|
| A | Acids (Inorganic, Oxidizing) |
| B | Acids (Organic) |
| C | Bases (Alkalis) |
| D | Oxidizers |
| E | Solvents (Organic) |
| F | Water Reactive |

Returning to the flow-chart in fig 7, it is determined S73 whether or not the first product contained in the first tank 5a and the second product contained in the second tank 5b are chemically incompatible. Referring to the discussion related to fig 9 above, this determination may involve applying the set of chemical compatibility rules, such as the table 39 in fig 9, on the received chemical compatibility information of the second product in the second tank 5b, and the retrieved chemical compatibility information of the first product in the first tank 5a.

When the chemical compatibility rules indicate that a combination of, for example, the basic chemical properties A-F in fig 9 is not compatible, the method proceeds to provide S74 an indication that the first product contained in the first tank 5a and the second product contained in the second tank 5b are chemically incompatible. As is schematically indicated in fig 8, such an indication may be provided as a visual and/or audible indication by the tank monitoring device 9a of the first tank 5a. The tank monitoring device 9b of the second tank 5b may also provide an indication. Such an indication may result from performing the same procedure as described above by the tank monitoring device 9b of the second tank 5b. Alternatively, the tank monitoring device 9a of the first tank 5a may provide a signal to the tank monitoring device 9b of the second tank 5b, encoding that there is chemical incompatibility. Furthermore, as is schematically indicated in fig 8 by the "flash" between the tank monitoring device 9a of the first tank 5a and the cloud representing the asset management system 1, an indication of chemical incompatibility may also be provided to the asset management system 1. The indication of chemical incompatibility/compatibility may additionally include a unique identifier of the first tank 5a and/or of the second tank 5b. Further, the indication may include information about a position of at least one of the first tank 5a and the second tank 5b. The indication may also include information about the reason for the determined incompatibility. Such additional information may, for example, be useful as evidence of proper handling of the tanks 5a, 5b by the asset management system 1 and/or by the operator 11.

Alternatively, or in addition, the indication of chemical incompatibility may be provided wirelessly to a wireless operator device, such as a mobile phone of the operator 11. The indication may be provided from the asset management system 1, or directly from the tank monitoring device 9a of the first tank 5a or the tank monitoring device 9b of the second tank 5b. In response to receiving the indication, the wireless operator device may provide a signal indicative thereof - such as an operator alert notification.

According to the flow-chart in fig 7, the process ends when it is found that the first product in the first tank 5a and the second product in the second tank 5b are chemically compatible. This need not necessarily be the case. One or both of the tank monitoring devices 5a, 5b may provide an indication that the products are chemically compatible. This may have the benefit that the operator 11 and/or the asset management system 1 gets a confirmation that the chemical compatibility evaluation has taken place.

The person skilled in the art realizes that the present invention by no means is limited to the preferred embodiments described above. On the contrary, many modifications and variations are possible within the scope of the appended claims.

## Claims

1. A tank monitoring device (9a) for monitoring a first tank (5a) containing a first product, the tank monitoring device (9a) comprising:
a memory (24) configured to store chemical compatibility information about the first product contained in the first tank (5a);
wireless communication circuitry (25) for wireless communication; and
a controller (27) for controlling operation of the tank monitoring device (9a), the controller (27) being configured to:
receive, using the wireless communication circuitry (25), from a tank monitoring device (9b) monitoring a second tank (5b), chemical compatibility information about a second product contained in the second tank (5b);
retrieve, from the memory (24), the chemical compatibility information about the first product contained in the first tank (5a);
determine, based on the chemical compatibility information about the first product and the chemical compatibility information about the second product, that the first product and the second product are chemically incompatible; and
provide an indication that the first product contained in the first tank (5a) and the second product contained in the second tank (5b) are chemically incompatible.

2. The tank monitoring device (9a) according to claim 1, the wireless communication circuitry (25) comprising:
first wireless communication circuitry (29) configured to implement a first communication architecture adapted for relatively long range wireless communication; and
second wireless communication circuitry (31) configured to implement a second communication architecture adapted for relatively short range wireless communication.

3. The tank monitoring device (9a) according to claim 2, the controller (27) being configured to receive the chemical compatibility information about the second product contained in the second tank (5b) using the second wireless communication circuitry (31).

4. The tank monitoring device (9a) according to claim 2 or 3, the controller (27) being configured to:
transmit, using the second wireless communication circuitry (31), the chemical compatibility information about the first product contained in the first tank (5a).

5. The tank monitoring device (9a) according to claim 4, the controller (27) being configured to:
broadcast the chemical compatibility information about the first product contained in the first tank (5a) at least once every hour.

6. The tank monitoring device (9a) according to any one of claims 2 to 5, the controller (27) being configured to:
receive, from an asset management system (1) using the first wireless communication circuitry (29), the chemical compatibility information about the first product contained in the first tank (5a).

7. The tank monitoring device (9a) according to any one of claims 2 to 6, the controller (27) being configured to:
transmit, to the asset management system (1) using the first wireless communication circuitry (29), a message indicating that the first product contained in the first tank (5a) and the second product contained in the second tank (5b) are chemically incompatible.

8. The tank monitoring device (9a) according to any one of the preceding claims:
the memory (24) being configured to additionally store a set of chemical compatibility rules (39); and
the controller (27) being configured to determine that the first product and the second product are chemically incompatible additionally based on the set of chemical compatibility rules (39).

9. The tank monitoring device (9a) according to any one of the preceding claims, the controller (27) being configured to:
receive, using the wireless communication circuitry (25), a unique identifier of the second tank (5b); and
provide the indication that the first product contained in the first tank (5a) and the second product contained in the second tank (5b) are chemically incompatible, including the unique identifier of the second tank (5b).

10. The tank monitoring device (9a) according to any one of the preceding claims:
further comprising level sensing circuitry (23) for measuring the level of the first product contained in the first tank (5a);
the controller (27) being configured to:
control the level sensing circuitry (23) to measure the level of the first product; and
transmit, using the wireless communication circuitry (25), a message indicative of the measured level of the first product to an asset management system (1).

11. A method of evaluating a chemical compatibility of products contained in neighboring tanks, the method comprising:
receiving (S71), by wireless communication circuitry (25) of a first tank (5a) containing a first product, chemical compatibility information about a second product contained in a second tank (5b);
retrieving (S72), by processing circuitry (27) of the first tank (5a), chemical compatibility information about the first product contained in the first tank (5a) from a memory (24) of the first tank (5a);
determining (S73), by the processing circuitry (27), based on the chemical compatibility information about the first product and the chemical compatibility information about the second product, that the first product contained in the first tank (5a) and the second product contained in the second tank (5b) are chemically incompatible; and
providing (S74), by the processing circuitry (27), an indication that the first product contained in the first tank (5a) and the second product contained in the second tank (5b) are chemically incompatible.

12. The method according to claim 11, comprising:
receiving, by the wireless communication circuitry (25) of the first tank (5a), the chemical compatibility information about the second product contained in the second tank (5b) when the first tank (5a) is within a maximum range of corresponding wireless communication circuitry of the second tank (9b).

13. The method according to claim 11 or 12, comprising:
transmitting, by the wireless communication circuitry (25) of the first tank (5a), the chemical compatibility information about the first product contained by the first tank (5a).

14. The method according to claim 13, comprising
broadcasting the chemical compatibility information about the first product contained in the first tank (5a) at least once every hour.

15. The method according to any one of claims 11 to 14, wherein it is determined that the first product and the second product are chemically incompatible additionally based on a set of chemical compatibility rules (39) stored in the memory (24) of the tank.
